# EUROPEAN PATENT APPLICATION

(11) **EP 4 120 187 A1**
(43) Date of publication of application: **18.01.2023**
(21) Application number: 21186023.4
(22) Date of filing: 16.07.2021
(51) Int. Cl.: G06T 7/00, G06T 7/11

(54) **A METHOD FOR MEASURING A PROGNOSTIC MARKER IN PROSTATE CANCER**

(71) Applicant: Universitätsklinikum Hamburg-Eppendorf, 20246 Hamburg (DE)
(72) Inventor: Blessin, Niclas Christian, 20246 Hamburg (DE); Sauter, Guido, 22397 Hamburg (DE); Graefen, Markus, 20148 Hamburg (DE)
(74) Representative: Hauck Patentanwaltspartnerschaft mbB

(57) **Abstract**

A method for measuring a prognostic marker in prostate cancer, the method comprising the following steps:
a) providing a section of prostate tissue,
b) multiplex fluorescence IHC-staining of the tissue section using a first marker for labelling epithelial cells, a second marker for labelling basal cells and at least one prognostic marker, and obtaining corresponding multiplex fluorescence image data,
c) performing a first automatic image data analysis step of the multiplex fluorescence image data leading to segmented image data segmented according to cell types including at least epithelial cells and basal cells,
d) performing a second automatic image data analysis step of the segmented image data and/or the multiplex fluorescence image data identifying image regions comprising non-malignant cells, and excluding these image regions from further analysis, and
e) determining a quantitative parameter of the at least one prognostic marker for epithelial cells in an image region not excluded in the second image data analysis step.

## Description

Prostate cancer is the most prevalent cancer in men in Western societies. Although the majority of prostate cancers behave in an indolent manner, a small subset is highly aggressive and requires extensive treatment. The Gleason score is the strongest prognostic parameter, but its high interobserver variability is limiting its effectiveness, and it requires a time-consuming evaluation of tissue sections by experienced pathologists, which often is a bar to its application on a routine basis in clinical practice.

For this reason, various techniques for semi-automatic and automatic analysis of prostate tissue specimen have been discussed. For example, the publication "Epithelium segmentation using deep learning in H&E-stained prostate specimens with immunohistochemistry as reference standard" of Bulten, W., Bandi, P., Hoven, J. et al., Sci Rep 9, 864 (2019), https://doi.org/10.1038/s41598-018-37257-4, suggests a deep learning method to segment epithelial tissue in digitised hematoxylin and eosin (H&E) stained prostatectomy slides. Immunohistochemistry (IHC) was used as reference standard to create a ground truth compared to manual outlining on H&E slides. The authors state their system accurately segmented both intact glands and individual tumour epithelial cells. However, further analysis must be performed manually, and the study was based on brightfield H&E staining so that a continuous quantification of the prognosis marker intensity is not feasible. Bulten et al. published this work as a preparation for a deep learning-based Gleason Grading in H&E staining which has been published recently "Automated deep-learning system for Gleason grading of prostate cancer using biopsies: a diagnostic study" by Bulten et al., Lancet Oncology, 2020, https://pubmed.ncbi.nlm.nih.gov/31926805/.

The publication "Prevalence of CD8+ cytotoxic lymphocytes in human neoplasms" of Blessin, N.C., Spriestersbach, P., Li, W. et al., Cell Oncol. 43, 421-430 (2020), https://doi.org/10.1007/s13402-020-00496-7, describes an assessment of density of CD8⁺ lymphocytes in a range of different cancer types and subtypes. Tissue microarrays (TMA) were analyzed by CD8 immunohistochemistry followed by semi-automated image analysis of digitized slides. TMA spots were automatically identified and analyzed using a software to identify and annotate individual tissue spots. If necessary, the segmentation was corrected manually. Another software was used to determine the number of CD8⁺ cells in each tissue spot and to measure the area of each tissue spot.

Starting therefrom, it is an object of the invention to provide a method for measuring a prognostic marker in prostate cancer that can be incorporated in routine clinical practice and allows for a more reliable identification of aggressive prostate cancers.

This object is solved by the method of claim 1. Advantageous aspects are given in the dependent claims.

The method is for measuring a prognostic marker in prostate cancer and comprises the following steps:
a) providing a section of prostate tissue,
b) multiplex fluorescence IHC-staining of the tissue section using a first marker for labelling epithelial cells, a second marker for labelling basal cells and at least one prognostic marker, and obtaining corresponding multiplex fluorescence image data,
c) performing a first automatic image data analysis step of the multiplex fluorescence image data leading to image data segmented according to cell types including at least basal cells and epithelial cells,
d) performing a second automatic image data analysis step of the segmented image data and/or the multiplex fluorescence image data identifying image regions comprising non-malignant cells, and excluding these image regions from further analysis, and
e) determining a quantitative parameter of the at least one prognostic marker for epithelial cells in an image region not excluded in the second image data analysis step.

In the first step, step a), a section of prostate tissue is provided. This may be done for example by cutting a biopsy or prostatectomy specimen.

In the second step, step b), multiplex fluorescence IHC-staining of the tissue section and corresponding image data acquisition is carried out. Several fluorescent markers are used, namely a first marker for labelling epithelial cells, a second marker for labelling basal cells and at least one prognostic marker for labelling a prognostic marker expression profile. Digital images of the tissue section are obtained, in particular using an epifluorescence microscope. This can be done by obtaining any number of images, each including the information of one or more of the markers used. For example, the different markers can be recorded in a single image by using different wavelengths of fluorescent light. In the alternative, it is an option to gather the information on the different markers in a number of consecutive images after separate staining experiments carried out for the same tissue section. In the latter case, the images obtained may be combined to a single image including the information on all of the markers (e.g. by preserving an individual image layer for every stained IHC marker), or may be registered with each other for further analysis.

No matter how data is acquired and processed, the multiplex fluorescence image data provided for the following image analysis includes quantitative information with regard to all of the markers used.

Based on the multiplex fluorescence image data obtained, as a next step, step c), a first automatic image data analysis is carried out, leading to image data segmented according to cell types. The cell types included in the segmentation are at least epithelial cells and basal cells, which can be identified based on the expression patterns of the first and second marker, possibly in combination with additional, structural features of the image data. If desired, additional cell types and/or image regions may be identified as additional segments, such as image regions corresponding to stroma, autofluorescence or imaging artefacts. Step c) may be carried out in two consecutive steps, namely a first step c1) in which cell nuclei are identified, and a second step c2) in which for each cell nucleus, a surrounding cell is identified and assigned to a specific cell types. In this case, step c2) may apply an additional detection framework (e.g., deep learning based) to classify the staining of every stained marker and to assign the marker composition to a specific cell type. This additional detection framework may analyse, in addition to the staining, structural features such as cell shapes, etc.

This first image data analysis step, step c), may use any type of digital, automatic data processing. Preferably, the first image data analysis step is carried out fully automatically, without any human interaction. As a result of the first image data analysis step, segmented image data are available, which include defined pixels and/or pixel values and/or pixel regions corresponding to certain cell types.

As a second automatic image data analysis step, step d), the segmented image data and/or the multiplex fluorescence image data are further analysed to identify image regions comprising non-malignant cells. These image regions are excluded from further analysis. The purpose of this step is to focus the subsequent quantitative assessment of the at least one prognostic marker on the tumor cells. Preferably, the second image data analysis step is carried out fully automatically, without any human interaction.

In a final step, a quantitative parameter of the at least one prognostic marker for epithelial cells in an image region not excluded in the second image data analysis step is determined. This can be done fully automatically as well, based on the fluorescence intensities of the at least one prognostic marker. Ideally, the quantitative parameter refers to tumor cells only. The quantitative parameter is designed such that it provides valuable prognostic information, for example comparable to a Gleason grade. In the final step, information obtained in the first and second automatic image data analysis steps is combined. In this context, it should be noted that any quantitative information on the prognostic marker available only globally, such as for the entire image region or any other image region containing a mixture of different cell types and/or of non-malignant cells and tumor cells, is of little prognostic value. In accordance with the invention, however, it is possible to combine information on cell-types and information on prognostic marker intensities obtained from the same tissue section, based on multiplex fluorescence imaging, and to evaluate both types of information on a cell-level, for the very same cells. With other imaging techniques, in particular with bright-field microscopy and conventional staining techniques such as H&E-staining, this is not possible. At the same time, in accordance with the invention, only one single tissue section is required, which is a huge advantage whenever only small specimen are available, as is typically the case in biopsy procedures.

The inventors realized that when following these steps, important and reliable information on the prognosis of a patient with prostate cancer can be provided. An important advantage of the method is that all data needed to come to a conclusion is gathered from a single specimen using multiplex fluorescence immunohistochemistry. Quantitative prognostic information is obtained automatically, in particular without needing to re-assess a specimen after a pathologist has determined certain areas of interest. Another important advantage is that the method circumvents current difficulties emanating from inter-observer variability. For these reasons, the method is well-suited for clinical routine.

According to an aspect, in the second automatic image data analysis step, step d), distances between epithelial cells and basal cells are calculated and epithelial cells being closer than a predefined distance to a nearest basal call are identified as non-malignant. The predefined distance may be in a range from 5 µm to 60 µm, in particular in a range from 15 µm to 25 µm. This approach to distinguish healthy tissue from tumor tissue is based on the insight that in healthy gland tissue, epithelial cells are usually arranged in proximity to a layer of basal cells, whereas this structure is often destroyed in tumor tissue. Based on the outcome of the first automatic image data analysis step, that is on the segmented image data, a mathematical algorithm can, for each epithelial cell, calculate a distance to the nearest basal cell and then apply an upper limit to select epithelial cells that are likely non-malignant. The inventors found that this simple approach leads to very good results. This is true even if the upper limit applied is relatively large, in which case some malignant epithelial cells may be erroneously categorized as healthy, which is attributed to the fact that the subsequent quantitative information will deliver good prognostic information as long as the image region covered does not include too many healthy cells.

According to an aspect, in the second automatic image data analysis step, step d), a second deep learning system is applied comprising a convolutional neural network. Deep learning systems have recently found wide application in radiology, because they are available as standard software packages and can fulfil complex tasks. Following this approach, the second image data analysis step may in particular start from the original multiplex fluorescence image data, rather than on the segmented image data. This means the first and second automatic image data analysis steps need not be performed in any specific order. The results of both steps on the one hand provide a segmentation according to cell types, on the other hand according to non-malignant versus malignant tissue. Prior investigations showed that deep learning systems may distinguish malignant from non-malignant tissue based on H&E-stained, brightfield images. Here, based on the IHC-stained, multiplex fluorescent image data, superior precision can be achieved.

According to an aspect, the second deep learning system (which is applied in step d) is trained to classify the segmented image data and/or the multiplex fluorescence image data according to one or more of the following classes: benign gland, tumor gland, autofluorescence, stroma, and background. This helps to define the image regions to be excluded from further analysis with even greater precision.

According to an aspect, the first image data analysis step (step c) applies a first deep learning system comprising a convolutional neural network. In this case, the segmentation according to cells is done by a deep learning system, which system operates on meaningful input data acquired by multiplex fluorescent imaging. In general, one may also use a machine learning system, but deep learning was found to deliver better results.

According to an aspect, the first deep learning system (applied in step c/c2) carries out the segmentation according to cell types without an operator having to set a threshold value for an intensity of one of the markers. This means the multiplex fluorescence image data can be used as an input for the (fully automatic) first image data analysis step without any manual data processing. Defining a threshold value for an intensity of a marker, that is an intensity level above which the expression level of a target against which the respective marker is directed (background) is considered positive, is a difficult task because various factors such as the overall image brightness, signal-to-noise ratio, etc. need to be taken into account. For this reason, setting a suitable threshold is typically carried out manually by an experienced operator. The inventors realized that a suitably trained convolutional neural network does not rely on an operator setting a suitable threshold, which helps to further minimize inter-observer variability.

According to an aspect, the first deep learning system (which is applied in step c/c2) based on the multiplex fluorescence image data defines an individual threshold value for an intensity of at least one of the markers. In this aspect, it is still not necessary to provide additional input on the threshold value. Instead, the first deep learning system is trained to set at least one suitable threshold itself, in particular an individual threshold for each of the markers applied.

According to an aspect, the quantitative parameter of the at least one prognostic marker (which is determined in step e) is a ratio of the number of epithelial cells exhibiting a high fluorescence intensity of the at least one prognostic marker compared to the number of epithelial cells exhibiting a low fluorescence intensity of the at least one prognostic marker. In addition or in the alternative, the intensity (continuous fluorescence signal measurement) and/or the density (cells with high intensities of the at last one prognostic marker per square millimeter) of at least one prognostic marker can be quantified on automatically detected tumor cells. High and low fluorescence intensities may be determined based on a global reference defined for the entire tissue section or for a series of tissue sections arranged in a micro array. The mentioned ratio has been found to be of great prognostic value. However, other quantitative parameters may be used as well, in particular when evaluated on a cell-level.

According to an aspect, the at least one prognostic marker includes antibodies directed against proliferating cells. For example, the at least one prognostic marker may include Ki67 antibodies. Prognostic markers directed against proliferating cells provide important prognostic information, in particular when evaluated quantitatively on a cell-level, as explained above. However, any other prognostic markers directed against other proteins relevant for patient's outcome/response to therapy may also be used.

According to an aspect, at least two, at least three, at least five or at least ten different prognostic markers are applied.

According to an aspect, the first marker comprises a pan cytokeratin (CKpan) antibody, in particular AE1/AE3 antibodies. These markers are well-suited for labelling epithelial cells. Other markers can be also used for labelling epithelial cells, e.g., other cytokeratins, PSA, PSAP, PSMA, NKX3.1, EMA, vimentin, claudins, catherins.

According to an aspect, the second marker comprises a p63 antibody. This marker is well-suited for labelling basal cells. Other markers can be also used for labelling basal cells, e.g., MA903, D2-40, CK56, CK34betaE12.

According to an aspect, the multiplex fluorescence IHC-staining comprises diamidino-2-phenylindole (DAPI) staining. With DAPI-staining, cell nuclei can be labelled, which is helpful in particular for step c1) described above, that is for the first step of the segmentation according to cell types.

It is noted that the inventive method and all of its aspects described above can also be applied to other types of cancer, in particular to cancer of gland tissue, such as breast cancer. Other gland tissues than prostate tissue, in particular breast tissue, have a structure comparable to prostate tissue, comprising basal cells and epithelial cells. The entire disclosure of this application therefore relates as well to a method for measuring a prognostic marker in cancer of gland tissue and/or in breast cancer. The method then is applied to a section of gland tissue or breast tissue, respectively, instead of to a section of prostate tissue.

In the following, the invention is explained in greater detail based on the following figures.

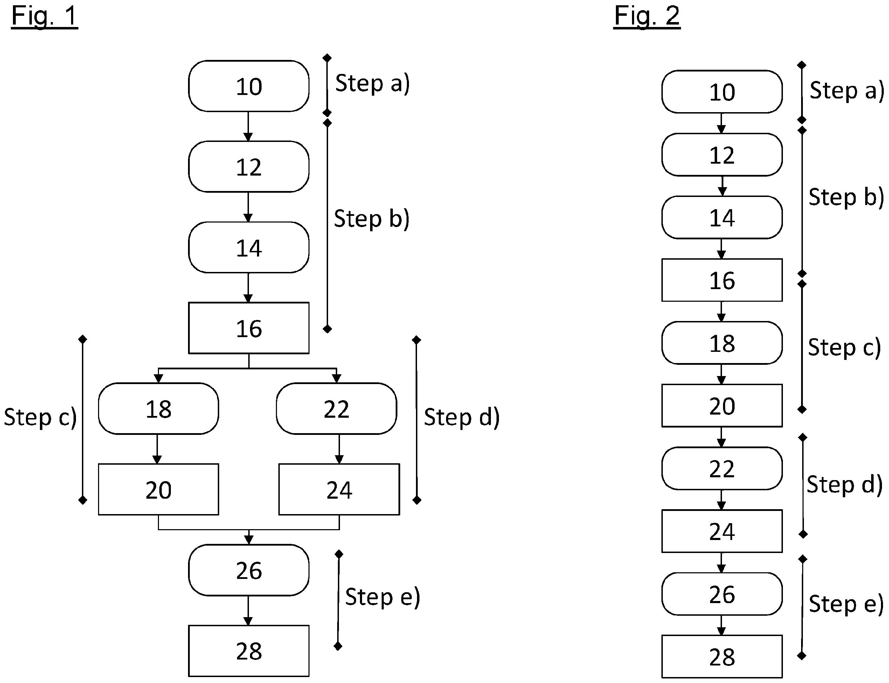

- Fig. 1: shows a flow diagram illustrating the steps of a first embodiment of the method;
- Fig. 2: shows a flow diagram illustrating the steps of a second embodiment of the method;

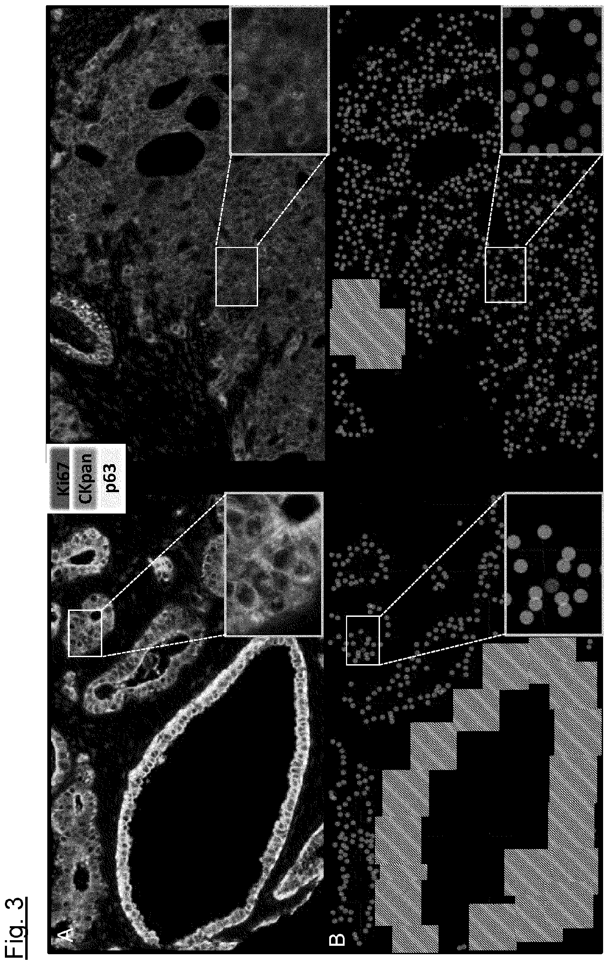

- Fig. 3:: illustrates of an approach for automated Ki67-LI assessment. Panel A shows representative images of predominantly healthy prostate glands (left) and predominantly prostate cancer (right) using multiplex fluorescence immunohistochemistry to identify CKpan⁺ epithelial cells (green), p63⁺ basal cells (white), and Ki67⁺ proliferating cells (red). The analysis approach used in this study combines a deep learning-based (U-Net) cell detection with cell-cell distance analysis. Panel B shows the results of the automated analysis approach. Prostate cancer cells were automatically included (light green and light red) and healthy normal glands (dark green, dark white) were excluded. A single dot represents the center of a single cell;

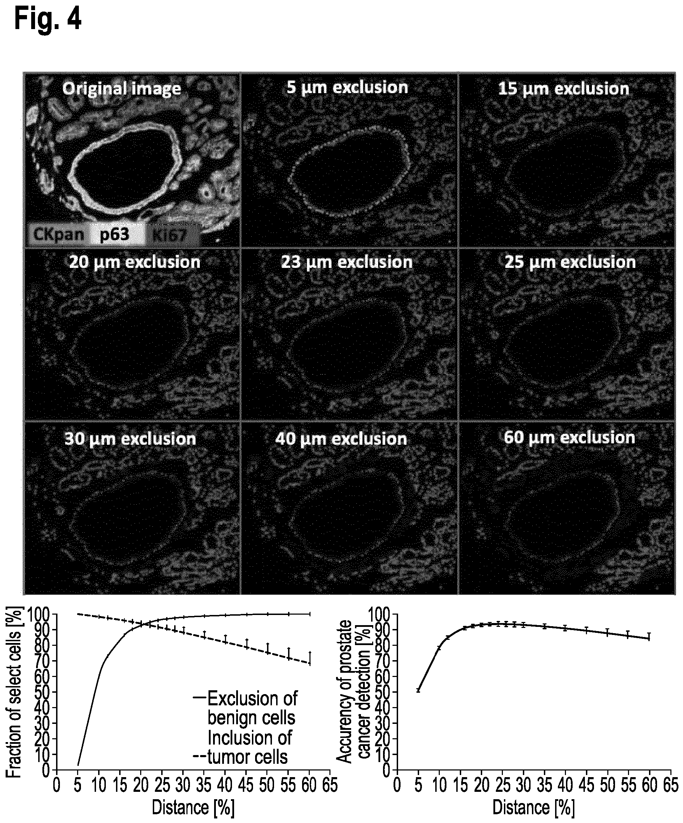

- Fig 4:: illustrates an automated prostate cancer detection by the combination of distance measurements and a deep learning-based cell detection (approach 1). A convolutional neural network (U-Net) for cell detection facilitated an additional distance measurement algorithm. The distance between a CKpan⁺ epithelial cell and the nearest p63⁺ basal cells was automatically measured. The highest accuracy of prostate cancer detection was achieved by excluding all epithelial cells at a ≤23µm distance to the nearest basal cell (benignant gland cells). For this purpose, 400 annotations of benign and malignant prostate glands were made by a pathologist containing a total of 262434 healthy and malignant cells; and

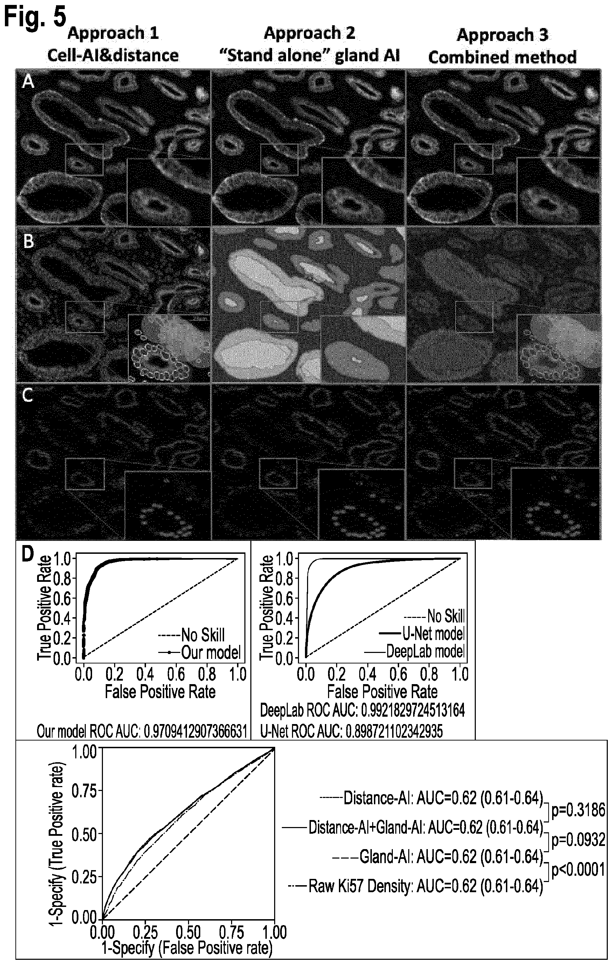

- Fig. 5:: compares the performance of three different approaches for automated prostate cancer detection. Approach 1 (cell detection deep learning system combined with distance analysis), approach 2 ("stand alone" gland detection deep learning system), and approach 3 (combination of approach 1 and 2) are shown by visualizing the classification performance in raw images (panel A and panel B) and the corresponding visualization of the calcification based on the output data (panel C). Approach 1-3 performed all significantly better than the raw Ki67-LI in 11845 patients (p<0.0001, panel D).

The method illustrated in Fig. 1 begins with a first step 10 in which a tissue section is prepared. For example, a section with a thickness of 4 µm is cut from a 0.6 mm diameter specimen obtained in a prostate biopsy procedure.

In the second step 12, multiplex fluorescence IHC staining is carried out, including at least a first marker for labelling epithelial cells, a second marker for labelling basal cells and at least one prognostic marker.

In the third step 14, multiplex fluorescence digital image data is obtained (scanning). As explained above, steps 12 and 14 may be repeated for one or more markers, until image data representing all relevant markers is available. As a result, multiplex fluorescence image data 16 is available for further analysis.

In the embodiment of Fig. 1, a first automatic image data analysis step 18 processes the multiplex fluorescence image data 16 and thereby produces segmented image data 20, that is image data segmented according to cell types including at least epithelial cells and basal cells. The first automatic image data analysis step 18 applies a first deep learning system comprising a convolutional neural network.

After, before or in parallel to the first automatic image data analysis step 18, a second automatic image data analysis step 22 is carried out. This step processes the multiplex fluorescence image data 16 and identifies image regions therein comprising non-malignant cells. The information on these image regions is represented by box 24. These image regions are excluded from further analysis.

In the embodiment of Fig. 1, the second automatic image data analysis step 22 applies a second deep learning system comprising a convolutional neural network, which is trained to classify the multiplex fluorescence image data 16 according to one or more of the following classes: benign gland, tumor gland, autofluorescence, stroma, and background.

The further step 26 is carried out after the first and second automatic image data analysis steps 18, 22 are completed. In this step, which processes the segmented image data 20 as well as the information 24 on image regions comprising non-malignant cells, a quantitative parameter 28 of the at least one prognostic marker for epithelial cells in an image region not excluded in the second image data analysis step 22 is determined. In step 26, the multiplex fluorescence image data 16 may be used as an additional input.

The quantitative parameter 28 of the at least one prognostic marker is a ratio of the number of epithelial cells exhibiting a high fluorescence intensity of the at least one prognostic marker compared to the number of epithelial cells exhibiting a low fluorescence intensity of the at least one prognostic marker.

In the method illustrated in Fig. 2, steps 10, 12, 14, and 18 as well as the corresponding results are the same as in Fig. 1. The second automatic image data analysis step 22, however, does not necessarily process the original multiplex fluorescence image data 16, but starts with the segmented image data 20. In the second automatic image data analysis step 22, distances between epithelial cells and basal cells are calculated and epithelial cells being closer than a predefined distance to a nearest basal call are identified as non-malignant. Hence, the information 24 on image regions comprising non-malignant cells is obtained with a different approach.

Step 26 then is similar to step 26 as described with referenced to Fig. 1 and once more leads to the desired quantitative parameter 28. Step 26 uses as input data both the segmented image data 20 and the information 24. In some cases, the original multiplex fluorescence image data 16 may be used as additional input.

In the following, a more detailed embodiment is described based on a large patient study using KI67 as a prognostic marker.

Ki67 is a nuclear antigen, which is expressed in proliferating cells from G1 to M-phase of the cell cycle. The quantification of Ki67 expression by conventional bright field immunohistochemistry, which is known as the Ki67 labelling index (Ki67-LI: number of Ki67⁺ cancer cells divided by all cancer cells), has been proven to be a strong prognostic parameter in prostate cancer. Earlier studies made use of several methods for KI67 quantification such as counting of Ki67⁺ cancer cells, semiquantitative scoring systems or semi-automated image analysis using pixel-based scores and nuclear quantification algorithms. The manual quantification of Ki67⁺ tumor cells is time consuming and cumbersome. Although, the computerized quantification of Ki67⁺ cancer cells has been studied extensively, as yet the automated differentiation between normal healthy prostate tissue and prostate cancer represents the most significant hurdle towards routine use. Accordingly, assessment of the Ki67-LI is currently not done in routine clinical practice.

To enable an automated assessment of the Ki67-LI, here we have developed an analysis approach to automatically differentiate tumor cells from benign cells for quantification of molecular features in prostate cancer. This approach incorporates a deep learning-based cell detection (referred to above as first automatic image data analysis step), which was combined with cell-cell distance analysis and multiplex fluorescence immunohistochemistry. The KI67-LI was automatically measured in a cohort of more than 10'000 prostate cancers to evaluate its potential clinical utility.

### Material and Methods

**Patients and tissues.** The study included prostatectomy specimens from 12524 consecutive patients, operated between 1993 and 2015 at the University Medical Center Hamburg-Eppendorf. Every single patient was represented by a tissue core, measuring 0.6mm in diameter, in a tissue microarray (TMA) format. An additional "heterogeneity" tissue microarray was constructed from 826 selected prostatectomy specimens where 3 to 6 different tumor foci were available for the individual patients. An experience pathologist (GS) quantitated the Gleason patterns based on which ISUP grades, quantitative Gleason grades and IQ Gleason scores were calculated, for each of the TMA spots of 826 patients included in the "heterogeneity" TMA.

**Multiplex fluorescence immunohistochemistry.** Freshly cut 4-µm consecutive tissue sections were used for multiplex fluorescence immunohistochemistry (IHC) staining. For fluorescence multiplex IHC, the OPAL dye kit (Cat. # NEL811001KT, AKOYA Biosciences, Menlo Park, California, United States) was used. The experimental procedure was performed according to the manufacturer's instructions (AKOYA). Slides were initially boiled in an autoclave (30 minutes at 100-120°C in pH9 buffer) for antigen retrieval. The antibody panel consisted of a pan cytokeratin (CKpan) antibody for epithelial cell detection (MSVA, rabbit recombinant, clone MSVA-000R, dilution 1:150), a p63 antibody for labeling basal cells (mouse monoclonal custom made, clone IPH-63, dilution 1:700), and a Ki67 antibody for detecting proliferating cells (MSVA, rabbit recombinant, clone MSVA-267R, dilution 1:50). These were combined with diamidino-2-phenylindole (DAPI) staining in each experiment. One circle of antibody staining included peroxidase blocking, application of the primary antibody, detection with a secondary HRP-conjugated antibody, fluorescence dye detection, and removal of the bound antibodies by microwave treatment (5 minutes at 100°C and 5 minutes at a mean temperature of 93°C). This cycle was repeated three times for the remaining antibodies. Slides were subsequently counterstained with DAPI and mounted in antifade solution.

**Deep learning-based automated prostate cancer detection.** Our detection system consisted of a convolutional neural network (U-Net) for cell detection, which we have combined with a cell-cell distance analysis to detect tumor cells (i.e., AE1/3⁺ epithelial cells) which were located ≥23µm away from p63⁺ basal cell. The U-Net cell segmentation system was trained on 3693 tissue samples from more than 100 different tumor entities. The optimal distance between tumor cells and p63⁺ was evaluated in a training set of 400 annotations of benign and malignant prostate glands (containing 262434 healthy and malignant cells). The U-Net based cell detection combined with a cell-cell distance analysis approach was compared with a second deep convolutional network (DeepLab3+) which was trained to classify the multiplex fluorescence images into one of the five classes: Benign gland, tumor gland, autofluorescence, stroma and background. Receiver operating characteristic curves (ROC) confirmed a significantly improved predictive performance of the Ki67-LI detected by both approaches compared to a raw Ki67-LI assessment (p<0.0001, Figure 5). Both deep learning systems were trained and validated using python version 3.8 and the Visiopharm software package (Hoersholm, Denmark).

**Deep learning system for cell detection (U-net)** A convolutional neural network (U-Net) was trained for the purpose of detecting DAPI⁺ cell nuclei. The provisional deep learning system for cell segmentation was trained on 3954 manually annotated prostate cancer cells for 16h. This "raw" deep learning system was then used to label a larger training set including more than 10 000 cells from 10 different tumor entities. The cell detection labels from this first AI were exported, manually corrected and used to train a new deep learning system on this larger training set. This procedure -using the manually corrected results of the previous U-Net to train a new AI based on the corrected AI results - was used to continuously increased the training set. The final deep learning system (U-Net) for cell detection was trained on 3693 tissue samples (more than 6 000 000 cells) from more than 100 different tumor entities using python version 3.8 and the Visiopharm software package (Hoersholm, Denmark).

**Deep learning system for cell detection (U-net) combined with cell-cell distance measurements for automated prostate cancer detection.** To enable an automated assessment of the Ki67-LI, an analysis approach was developed to detect prostate cancer by including prostate cancer glands and excluding benignant gland. Our detection system consisted of a convolutional neural network (U-Net) based algorithm for cell detection, which was used to measure the intensity of AE1/3 and p63 on every individual cell. By using multiplex fluorescence IHC AE1/3⁺ epithelial cells and p63⁺ basal cells were detected. To evaluate whether the AE1/3⁺ epithelial cells belong to a benign or malignant gland the nearest distance to p63⁺ basal cell was calculated. Analyzing adjacent benignant and malignant glands by using different distances (ranging from 0 to 60µm) as a threshold for benign cell exclusion revealed that a distance of 23µm enabled the optimal fraction of correctly detected prostate cancer cells (94%, Figure 4). The areas under receiver operating characteristic curves revealed that the Ki67-LI measured by this U-net approach showed the highest AUC (AUC:0.62[0.61-0.64]) and the predictive performance was significantly better compared to a raw KI67 density (number of Ki67+ cells/ mm²) assessment (p<0.0001, Figure 5).

**Analysis of multiplex fluorescence IHC images.** Digital images of mfIHC stained slides were acquired with a Leica Aperio VERSA 8 automated epifluorescence microscope and a Zeiss Axio Scan.Z1 slide scanner. Image analysis was performed using the pretrained deep learning- based (U-net) approach for cell detection, cell segmentation, intensity measurement of the used fluorophores (range 0-255, i.e., a continuous numerical value indicating the fluorescence signal strength), processing the intensity values and cell-cell distance analysis using python version 3.8, R version 3.6.1 (The R foundation) and the Visiopharm software package (Hoersholm, Denmark). The intensity of each fluorochrome was recorded as raw intensity for the individual cells. Only staining intensities exceeding a predefined threshold were considered "positive". The threshold was selected in a multi-step procedure for each marker (Ki67, p63, AE1/3) according to the following procedure: The raw intensity values of all markers were plotted for pairwise comparison in 2D/ 3D scatter plots and 2D/ 3D t-distributed stochastic neighbor embedding (t-SNE) with logarithmic normalization using R (R "Rtsne" and "plotly" package) to perform a gating step (i.e., identification of homogenous cell populations that share a particular function), which is well known from flow cytometry. The semi-automatically acquired provisional cut-off values were reviewed via histopathological examination and via measuring the fluorescence intensity of each marker - in multiple regions of 50 to 200 cells with expected lack of expression - to compare the value of the cell with highest "false positive" measurement with the provisional cutoff values. The measured threshold from these validation steps was reviewed by a trained pathologist across multiple tissue types in a test-TMA before the cut-off has been applied for the whole study. The image analysis workflow has been described in details previously.

**Statistical Analysis.** Statistical calculations were performed with JMP Pro 15 software package (SAS Institute Inc., NC, USA) and R version 3.6.1 (The R foundation). The clinico-histopathological parameters of the patients were reported as counts and percentages for categorical data and compared using likelihood ratio chi-square tests. To estimate the prognostic value of the automated Ki67-LI, Kaplan-Meier Estimates (R "survival" package) were used for biochemical recurrence after radical prostatectomy as the study endpoint. The log-rank test was applied to assess differences between groups in Kaplan-Meier Estimates. To estimate whether the prognostic impact of the Ki67-LI was independent from pre- and postoperative parameters, multivariable Cox proportional hazard models were calculated. Time-dependent areas under receiver operating characteristic curves were used to estimate the predictive performance of the Ki67-LI (R "riskRegression" package). All p-values were two-sided, and p-values <0.05 were considered as significant.

### Results

**Technical aspects.** A total of 11845 (95%) of 12524 prostatectomy specimens in the "classical" tissue microarray (TMA) format (a single sample from each patient) and 630 of 826 patients (2876 of 4188 tumor samples) in the "heterogeneity" TMA (3 to 6 samples from each patient) were interpretable in this study. The remaining 1985 tumor samples were excluded due to the lack of representative cancer cells which were detected by using the combined approach of deep learning-based cell segmentation and automated cell-cell distance analysis (Figures 3, 4 and 5).

**Classical prognosis TMA analysis.** The mean Ki67-LI in 11845 evaluable cases was 4.56%. A high Ki67-LI was significantly linked to high Gleason grade, advanced pT stage, nodal metastasis, high preoperative PSA levels, and PSA recurrence. The risk for PSA recurrence increased continuously along with an increasing Ki67-LI, irrespective of how subgroups were formed (p<0.0001). Four multivariate Cox regression models including preoperatively (i.e., Gleason grade biopsy, clinical stage, preoperative PSA levels) and postoperatively available parameters (i.e., Gleason grade prostatectomy, pT-Stage, R-Stage, N-Stage) revealed that the Ki67-LI was an independent risk factor in all models (p<0.0001 each). The utility of the Ki67-LI measurement in addition to Gleason parameters was also demonstrated in receiver operating characteristic curves (ROC). These data showed that the area under the curve (AUC) increased from classical ISUP grades (AUC: 0.76) to IQ Gleason (AUC: 0.81 [p<0.0001]) and that the Ki67-LI added significant additional prognostic information in case of classical ISUP grades (AUC: 0.78 [p<0.0001]) but not in case of the quantitative Gleason grade or IQ Gleason (AUC: 0.81 [p=0.1245]). The strong prognostic role of the Ki67-LI was also shown by its prognostic relevance in all groups with identical Gleason grades of biopsy specimen (p≤0.0001 each), in four of six groups with identical Gleason grades of prostatectomy specimen (p≤0.015 each) and in 5 of 11 subgroups with identical quantitative Gleason scores (p≤0.023 each).

**Heterogeneity prognosis TMA analysis.** Assessing the Ki67-LI in up to 6 foci for 630 patients revealed a considerable heterogeneity (mean Ki67-LI range across all foci for a single patient 9.99±11.52%). Both the Ki67-LI and the Gleason grade obtained from these TMA spots were linked to the risk of PSA recurrence (p<0.0001 each). For the Ki67-LI, ROC curves showed that the predictive performance of both the "mean" and the "maximum" Ki67-LI increased similarly along with the number of foci analyzed per patient (single foci AUC: 0.63 vs. mean of six foci AUC:0.71 [p=0.0058]). Remarkably, the Ki67-LI obtained from the tissue spot with the highest Gleason score (AUC: 0.68) was similarly predictive as the mean (AUC:0.71 [p=0.240]) or maximum (AUC:0.71 [p=0.314]) Ki67-LI obtained from all 6 different tumor foci. ROC curves describing the predictive performance of the Ki67-LI in addition to the Gleason grades were prepared. These data confirmed that the AUC improved from the classical ISUP grade (AUC: 0.79) to the IQ Gleason (AUC:0.82 [p=0.0061]). The Ki67-LI added significant additional prognostic information to both the classical ISUP grade (AUC: 0.82 [p=0.0021]) and the IQ Gleason (AUC: 0.83 [p=0.0179]).

### Discussion

Although the prognostic role of tumor cell proliferation is intuitive and well established and despite of a strong need for better prognostic information in many cases of newly diagnosed prostate cancer, proliferation measurement has not become a routine procedure in clinical praxis. This is probably due to the cumbersome nature of precisely measuring the percentage of Ki67 immunostained tumor cells and the unsolved issue which biopsy (or biopsies) should be analyzed in case of multiple cancer containing diagnostic biopsies. To enable a speedy, automated, non-interactive method for Ki67 quantification, a multiplex fluorescence IHC approach was developed that utilizes deep learning-based cell detection and cell-cell distance measurements. Our approach for automated cancer detection mimicked the way pathologists use for identification of cancer cells and identified epithelial cells (CKpan positive) that were non-adjacent to basal cells (p63 positive). The efficiency of our approach for the quantification of Ki67⁺CKpan⁺ cells that were located ≥23µm away from the nearest p63⁺ basal cell was validated by the concordance of our data with previous studies, and the striking prognostic impact of the automatically determined Ki67-LI.

The successful assessment of the Ki67-LI in more than 10'000 prostate cancers revealed an average Ki67-LI of 4.6%. This is in the range of previous studies reporting an average Ki67-LI of primary prostate cancers ranging from 2.1% to 6.3% by using a wide range of Ki67 quantification methods on cohorts of 96 to 1004 prostate cancers. The large number of cases analyzed in this study enabled us to demonstrate that the prognostic impact of the Ki67-LI was independent of the selected cutoff levels and that the likelihood of PSA recurrence showed an almost linear relationship with the Ki67-LI. Moreover, the Ki67-LI provided independent prognostic information irrespective of whether it was compared with other prognostic parameters which were available in the preoperative or postoperative situation. These findings are in line with numerous previous studies that have shown a prognostic significance of tumor cell proliferation in prostate cancer by the assessment of Ki67-LI using conventional bright-field immunohistochemestry, DNA flow cytometry, or quantitative RT-PCR. It is of note that RNA based molecular panel tests that have been proposed for prostate cancer prognosis assessment essentially analyze proliferation associated genes.

Based on a unique database containing detailed histopathological and clinical outcome information from more than 20,000 prostate cancer patients, our group had shown that the prognostic information derived from the analysis of the Gleason patterns can be markedly improved by considering the percentage of Gleason 4 pattern within "Gleason 7" cancers and by further integrating Gleason 5 patterns in a continuous score. As these Gleason data were obtained through the analysis of an entire prostatectomy specimen it is obvious that the Gleason data of our prognosis TMA are far more representative of the entire tumor mass than the Ki67-LI obtained on a small 0.6mm TMA spot. The fact that the KI67-LI still provided strong additional prognostic significance within each of the traditional Gleason grade groups (ISUP) is indicative of the strong prognostic power of the KI67-LI. The fact that the Ki67-LI failed to provide additional prognostic impact in combination with the quantitative Gleason or IQ Gleason reflects the marked prognostic potential of a detailed morphological prostate cancer assessment.

The prognosis TMA utilized in this study contained one 0.6mm tumor sample per patient. This tumor quantity comes close to that often seen in diagnostic biopsies. TMAs are thus a suitable model to assess the potential prognostic utility of molecular markers in prostate needle biopsies. To further simulate the situation of multiple tumor-positive biopsies, an additional tumor "heterogeneity" TMA had been constructed that contained 3 to 6 samples per patient. To model the real-life situation of a biopsy as closely as possible, all samples of one patient were taken from different tumor containing tissue blocks and the number of samples was proportionate to the tumor diameter. The thorough analysis of the Gleason score by an experienced pathologist (GS) for every TMA spot of the heterogeneity TMA now enabled a fair evaluation of the potential additive value of the KI67-LI analysis as compared to histology alone because both parameters were obtained on identical tissues. The fact that the prognostic impact of both the "mean" and "maximum" Ki67-LI increased with the number of analyzed samples per patient reflects the increased representativity of the Ki67-LI assessment in larger tissues and is consistent with earlier data from the literature. Thus, the selective analysis of the tissue spot with the worst histology per patient resulted in a similar area under the curve as the "maximum Ki67-LI" or the "mean Ki67-LI" and also had a comparable predictive performance is potentially of high clinical significance. It suggests that the Ki67-LI assessment of the worst biopsy would be sufficient and result in the most representative prognostic value for prostate cancer patients. The fact that the Ki67-LI provided additional prognostic information for all Gleason reporting methods including ISUP grades and IQ Gleason scores underlines that assessing the Ki67-LI could be clinically important in the preoperative setting.

In conclusion, our data shows that the Ki67-LI is a strong and independent prognostic parameter in prostate cancer, which can rapidly and reproducibly be analyzed by artificial intelligence supported multiplex fluorescence immunohistochemistry. The major advantage of this method is the limitation of the analysis to tumor cells, which cannot be achieved in RNA or DNA based panel analyses. Further improvements of multiplex fluorescence immunohistochemistry will soon enable the simultaneous analysis of 8, 40 and more antibodies on one tissue section. The combination of Ki6-LI with other prognostic biomarkers holds great potential for powerful prognostic prostate cancer assays in the not-too-distant future.

## Claims

1. A method for measuring a prognostic marker in prostate cancer, the method comprising the following steps:
a) providing (10) a section of prostate tissue,
b) multiplex fluorescence IHC-staining (12) of the tissue section using a first marker for labelling epithelial cells, a second marker for labelling basal cells and at least one prognostic marker, and obtaining (14) corresponding multiplex fluorescence image data (16),
c) performing a first automatic image data analysis step (18) of the multiplex fluorescence image data (16) leading to segmented image data (20) segmented according to cell types including at least epithelial cells and basal cells,
d) performing a second automatic image data analysis step (22) of the segmented image data (20) and/or the multiplex fluorescence image data (16) identifying image regions comprising non-malignant cells, and excluding these image regions from further analysis, and
e) determining (26) a quantitative parameter (28) of the at least one prognostic marker for epithelial cells in an image region not excluded in the second image data analysis step (22).

2. The method of claim 1, wherein in the second automatic image data analysis step (22), distances between epithelial cells and basal cells are calculated and epithelial cells being closer than a predefined distance to a nearest basal call are identified as non-malignant.

3. The method of claim 2, wherein the predefined distance is in a range from 5 µm to 60 µm, in particular in a range from 10 µm to 25 µm.

4. The method of any of the claims 1 to 3, wherein in the second automatic image data analysis step (22), a second deep learning system is applied comprising a convolutional neural network.

5. The method of claim 4, wherein the second deep learning system is trained to classify the segmented image data (20) and/or the multiplex fluorescence image data (16) according to one or more of the following classes: benign gland, tumor gland, autofluorescence, stroma, and background.

6. The method of any of the claims 1 to 5, wherein the first automatic image data analysis step (18) applies a first deep learning system comprising a convolutional neural network.

7. The method of any of the claims 1 to 6, wherein the first deep learning system carries out the segmentation according to cell types without an operator having to set a threshold value for an intensity of one of the markers.

8. The method of any of the claims 1 to 7, wherein the first deep learning system based on the multiplex fluorescence image data defines an individual threshold value for an intensity of at least one of the markers.

9. The method of any of the claims 1 to 8, wherein the quantitative parameter (28) of the at least one prognostic marker is a ratio of the number of epithelial cells exhibiting a high fluorescence intensity of the at least one prognostic marker compared to the number of epithelial cells exhibiting a low fluorescence intensity of the at least one prognostic marker.

10. The method of any of the claims 1 to 9, wherein the at least one prognostic marker includes antibodies directed against proliferating cells.

11. The method of any of the claims 1 to 10, wherein the at least one prognostic marker includes Ki67 antibodies.

12. The method of any of the claims 1 to 11, wherein at least two, at least three or at least five different prognostic markers are applied.

13. The method of any of the claims 1 to 12, wherein the first marker comprises a pan cytokeratin (CKpan) antibody, in particular AE1/AE3 antibodies.

14. The method of any of the claims 1 to 13, wherein the second marker comprises a p63 antibody.

15. The method of any of the claims 1 to 14, wherein the multiplex fluorescence IHC-staining (12) comprises diamidino-2-phenylindole (DAPI) staining.
